# EUROPEAN PATENT SPECIFICATION

(11) **EP 4 125 980 B1**
(45) Date of publication and mention of the grant of the patent: **26.11.2025**
(21) Application number: 21715336.0
(22) Date of filing: 25.03.2021
(51) Int. Cl.: A61K 36/23, A61K 36/53, A61K 8/97, A61P 29/00

(54) **COMPOSITIONS COMPRISING CARUM CARVI AND ROSMARINUS OFFICINALIS EXTRACTS FOR USE IN TREATING INFLAMMATORY DISORDERS**
ZUSAMMENSETZUNGEN MIT CARUM CARVI- UND ROSMARINUS-OFFICINALIS-EXTRAKTEN ZUR VERWENDUNG IN DER BEHANDLUNG VON ENTZÜNDLICHEN ERKRANKUNGEN
COMPOSITIONS COMPRENANT DES EXTRAITS DE CARUM CARVI ET DE ROSMARINUS OFFICINALIS POUR L'UTLISATIONS DANS LE TRAITEMENT DES MALADIES INFLAMMATOIRES

(30) Priority: 26.03.2020 US 202063000402 P
(43) Date of publication of application: 08.02.2023
(73) Proprietor: Kenvue Brands LLC, Summit, NJ 07901 (US)
(72) Inventor: MAHMOOD, Khalid, Skillman, New Jersey 08558 (US); BRUN, Cecilia, 27100 Val De Reuil (FR); VAN DEN HEUVEL, Antonius P., J., Skillman, NJ 08558 (US); SERRANO, Jose, 27100 Val De Reuil (FR)
(74) Representative: Carpmaels & Ransford LLP
(86) International application number: PCT/IB2021/052490
(87) International publication number: WO 2021/191840

(56) References cited:
- EP-A1- 1 743 620
- EP-A1- 2 949 362
- WO-A1-2011/068813
- CN-A- 104 013 784
- US-A1- 2018 064 775

## Description

### FIELD

The present invention relates to compositions for the topical reduction of inflammation. More specifically, the present invention relates to compositions comprising extracts of *Carum carvi* seed and *Rosmarinus officinalis* leaf for treating or preventing inflammatory disorders.

### BACKGROUND

A wide variety of skin care actives suitable for use in personal care compositions are known. Examples of such skin care actives include olive leaf extract, which has been described as exhibiting antihypertensive and hypoglycemic activities, antiradical properties for alimentary and cosmetics, and anti-inflammatory activity when given via the oral route (see, for example, Leaf extract of Olea europea rich in oleuropeine, products from it, their application as medicines and compositions containing them. Combes, Georges; Escaut, Alexandre. Fr. Demande, FR 2507477 A1 19821217, 1982; Gonzalez M, et al, Hypoglycemic activity of olive leaf, Planta Med December 1992; 58(6):513-515; Use of an extract from the leaves of Olea Europea as an antiradical agent. Amari, Giorgio. Eur. Pat. Appl. (1999), EP 937455 A1 19990825; Fehri B, et al. Olea europaea L.: stimulant, anti-ulcer and anti-inflammatory effects. Boll Chim Farm (1996) 135(1): 42-49), Sigesbeckia (Holy Herb), which has been used as a remedy for ague, rheumatism, renal colic, and as a cure for ringworm in conjunction with glycerin, Lignum Sappan (Sappan Wood), which has been used to promote blood circulation and remove blood stasis, and to cause subsidence of swelling and relieve pain, and Feverfew, recognized as having significant medicinal properties when taken orally and used as a general febrifuge. Other skin care actives include oil extracts a such as Boswellia Serrata oil extract (Frankincense), described as exhibiting anti-tumor and anti-arthritic properties, and oat oil extract, described as exhibiting anti-irritant and an antioxidant properties.

US2018/0064775 relates to antimicrobial, antiviral and antifungal compositions and methods of use thereof.

EP1743620 relates to a hair cosmetic composition which is said to be "capable of treating hairs without causing damages to hairs and injuries of skins and having oxidation fixing effect of permanent wave and dyeing effect equal to or superior to those in a usual case".

EP2949362 relates to an AGEs-degrading agent, which contains at least one kind of plant extract obtained from a plant selected from the group consisting of peanut, rosemary, knotweed, leaf lettuce, sweet basil, thyme, spearmint, lemon balm, shiso, and rhubarb.

WO2011/068813 relates to compositions comprising a combination of extracts, and methods of preparing and using the same.

CN104013784 relates to a medicament for treating pelvic inflammation.

There is an ongoing need to develop combinations of skin care actives to effectively treat, reduce, and/or prevent inflammation of the skin due to any of a variety of sources. While there may be a variety of actives that tend to exhibit anti-inflammatory properties, it is generally difficult, if not impossible, to predict combinations of such actives that will exhibit unexpectedly high, synergistic anti-inflammatory properties and effectiveness at reducing inflammation.

In addition, in certain uses it may be desirable to use relatively low amounts of actives from a cost perspective, to reduce undesirable color or odor associated with certain actives, for stability reasons, and the like. However, many actives, or combinations thereof, tend to exhibit relatively little or no effectiveness at reducing inflammation when applied in relatively low amounts. Accordingly, there is an ongoing need for combinations of skin care actives that exhibit relatively high, synergistic anti-inflammatory properties and effectiveness, and certain combinations that may further be effective even in relatively low amounts.

### SUMMARY OF THE INVENTION

The present invention is defined by the claims.

Accordingly, one aspect of the invention pertains to a composition for use in a method of treating or preventing inflammatory disorders, the method comprising: administering to a patient in need thereof a therapeutically effective dose of the composition, wherein the composition comprises *Carum carvi* seed extract and *Rosmarinus officinalis* extract, wherein the *Carum carvi* seed extract is present at a concentration of about 50 to about 350 µg/mL, and the *Rosmarinus officinalis* extract is a *Rosmarinus officinalis* leaf extract present at a concentration of about 0.5 to about 25 µg/mL; wherein the *Carum carvi* seed extract and *Rosmarinus officinalis* leaf extract each independently comprise polar extracts; and wherein the polar extract was extracted using one or more of C₁-C₈ alcohols, C₁-C₈ polyols, C₁-C₈ glycols and combinations of two or more thereof.

In one or more embodiments, the composition further comprises a material selected from the group consisting of surfactants, chelating agents, emollients, humectants, conditioners, preservatives, opacifiers, fragrances, and combinations of two or more thereof. The *Carum carvi* seed extract and *Rosmarinus officinalis* extract each independently comprise a polar extract. In further embodiments, both the *Carum carvi* seed extract and *Rosmarinus officinalis* extract comprise polar extracts. In one or more embodiments, the polar extract was extracted with one or more solvents selected from the group consisting of C₁-C₈ alcohols, C₁-C₈ polyols, C₁-C₈ glycols and combinations thereof. In some embodiments, the *Carum carvi* seed extract and *Rosmarinus officinalis* extract each independently comprise one or more solvents comprising ethanol and water. In one or more embodiments, one or both of the *Carum carvi* seed extract and *Rosmarinus officinalis* extract are substantially free of nonpolar extracts. In some embodiments, one or both of the *Carum carvi* seed extract and *Rosmarinus officinalis* extract are substantially free of essential oils. In one or more embodiments, the composition is in the form of a tablet, pill, or capsule. In some embodiments, the composition is in the form of a solution, suspension, emulsion, lotion, cream, serum, gel, stick, spray, ointment, liquid wash, soap bar, shampoo, hair conditioner, paste, foam, powder, mousse, shaving cream, hydrogel, or film-forming product. In one or more embodiments, the *Carum carvi* seed extract and *Rosmarinus officinalis* extract are present in the composition at a combined concentration of about 0.1 to about 20 wt.% by weight of the total composition. In some embodiments, the *Carum carvi* seed extract and *Rosmarinus officinalis* extract are present at a ratio of about 1:2 to about 1:100. The *Carum carvi* seed extract is present at a concentration of about 50 to about 350 µg/mL, and the *Rosmarinus officinalis* extract is a *Rosmarinus officinalis* leaf extract present at a concentration of about 0.5 to about 25 µg/mL. In some embodiments, the *Carum carvi* seed extract is present at a concentration of about 50 µg/mL, and the *Rosmarinus officinalis* leaf extract is present at a concentration of about 0.5 to about 5 µg/mL.

In one or more embodiments, the inflammation comprises CCR2 receptor-mediated inflammation. The *Carum carvi* seed extract and *Rosmarinus officinalis* leaf extract each independently comprise a polar extract. In further embodiments, both the *Carum carvi* seed extract and *Rosmarinus officinalis* extract comprise polar extracts. In one or more embodiments, the polar extract was extracted with one or more solvents selected from the group consisting of C₁-C₈ alcohols, C₁-C₈ polyols, C₁-C₈ glycols and combinations thereof. In some embodiments, one or both of the *Carum carvi* seed extract and *Rosmarinus officinalis* leaf extract are substantially free of nonpolar extracts. In one or more embodiments, one or both of the *Carum carvi* seed extract and *Rosmarinus officinalis* leaf extract are substantially free of essential oils. In some embodiments, the composition is applied to a surface of skin of the patient. In one or more embodiments, the composition is applied to a mucous membrane of the patient. In some embodiments, the composition is administered in the form of a solution, suspension, lotion, cream, serum, gel, stick, spray, ointment, liquid wash, soap bar, shampoo, hair conditioner, paste, foam, powder, mousse, shaving cream, hydrogel, or film-forming product. In one or more embodiments, the composition is administered orally. In some embodiments, the composition is administered in the form of a tablet, pill, capsule. In one or more embodiments, the *Carum carvi* seed extract and *Rosmarinus officinalis* extract are present in the composition at a combined concentration of about 0.1 to about 20 wt.% by weight of the total composition. In some embodiments, the *Carum carvi* seed extract and *Rosmarinus officinalis* extract are present at a ratio of about 1:2 to about 1:100. The *Carum carvi* seed extract is present at a concentration of about 50 to about 350 µg/mL, and the *Rosmarinus officinalis* extract is a *Rosmarinus officinalis* leaf extract present at a concentration of about 0.5 to about 25 µg/mL. In some embodiments, the *Carum carvi* seed extract is present at a concentration of about 50 µg/mL, and the *Rosmarinus officinalis* leaf extract is present at a concentration of about 0.5 to about 5 µg/mL.

Yet another aspect of the invention pertains to a composition comprising:
Carum carvi seed extract at a concentration of about 50 to about 350 µg/mL;
Rosmarinus officinalis leaf extract at a concentration of about 0.5 to about 25 µg/mL; and
a pharmaceutically and/or cosmetically acceptable carrier,
wherein the *Carum carvi* seed extract and *Rosmarinus officinalis* leaf extract are aqueous alcohol extracts, and wherein the composition is in the form of a solution, suspension, emulsion, lotion, cream, serum, gel, stick, spray, ointment, liquid wash, soap bar, shampoo, hair conditioner, paste, foam, powder, mousse, shaving cream, hydrogel, or film-forming product. In some embodiments, the *Carum carvi* seed extract is present at a concentration of about 50 µg/mL, and the *Rosmarinus officinalis* leaf extract is present at a concentration of about 0.5 to about 5 µg/mL.

These and other features and advantages of the present invention will be readily apparent from the following detailed description of the invention.

### DETAILED DESCRIPTION OF THE INVENTION

As used herein, "skin in need of reducing skin inflammation" means a portion or region of a skin of a patient exhibiting redness or erythema, edema, or being reactive or sensitive to external elements. External elements include, but are not limited to, sun rays (UV, visible, IR), microorganisms, atmospheric pollutants such as ozone, exhaust pollutants, chlorine and chlorine generating compounds, cigarette smoke, cold temperature, heat, soaps and detergents, cosmetics, jewelry. Inflammatory disorders and related conditions which may be treated or prevented by use of the compositions of this invention include, but are not limited to the following: arthritis, bronchitis, contact dermatitis, atopic dermatitis, psoriasis, seborrheic dermatitis, sumac and poison oak dermatitis, eczema, allergic dermatitis, polymorphous light eruptions, inflammatory dermatoses, folliculitis, alopecia, poison ivy, insect bites, acne inflammation, irritation induced by extrinsic factors including, but not limited to, chemicals, trauma, pollutants (such as cigarette smoke) and sun exposure, secondary conditions resulting from inflammation including but not limited to xerosis, hyperkeratosis, pruritus, post-inflammatory hyperpigmentation, scarring and the like. In one or more embodiments, the inflammatory disorders and related conditions which may be treated or prevented using the methods of the invention are arthritis, inflammatory dermatoses, contact dermatitis, allergic dermatitis, atopic dermatitis, polymorphous light eruptions, irritation, including erythema induced by extrinsic factors, acne inflammation, psoriasis, seborrheic dermatitis, eczema, poison ivy, poison oak, poison sumac, insect bites, folliculitis, alopecia, and secondary conditions and the like.

As used herein, "cosmetically / dermatologically acceptable" means suitable for use in contact with tissues (e.g., the skin or hair) without undue toxicity, incompatibility, instability, irritation, and/or allergic response, and the like.

As used herein, the term "safe and effective amount" means an amount sufficient to induce the desired effect, but low enough to avoid serious side effects. The safe and effective amount of the compound, extract, or composition will vary with, e.g., the age, health and environmental exposure of the end user, the duration and nature of the treatment, the specific extract, ingredient, or composition employed, the particular pharmaceutically-acceptable carrier utilized, and like factors.

As used herein, "essential oil" refers to a distillation product obtained from a plant, preferentially via steam distillation, which contains the characteristic fragrance of the plant or specific part of the plant from which it is obtained. In some embodiments, the essential oil is a concentrated hydrophobic liquid obtained from a plant which contains a mixture of volatile oils (including e.g., volatile aroma compounds).

As used herein, "essentially free" or "substantially free" of an ingredient means containing less than 0.1 weight percent, or less than 0.01 weight percent, or none of an ingredient.

To provide a more concise description, some of the quantitative expressions given herein are not qualified with the term "about". It is understood that whether the term "about" is used explicitly or not, every quantity given herein is meant to refer to the actual given value, and it is also meant to refer to the approximation to such given value that would reasonably be inferred based on the ordinary skill in the art, including approximations due to the experimental and/or measurement conditions for such given value.

To provide a more concise description, some of the quantitative expressions herein are recited as a range from about amount X to about amount Y. It is understood that wherein a range is recited, the range is not limited to the recited upper and lower bounds, but rather includes the full range from about amount X through about amount Y, or any amount or range therein.

Accordingly, one aspect of the invention pertains to a composition comprising: *Carum carvi* seed extract; *Rosmarinus officinalis* extract; and a pharmaceutically and/or cosmetically acceptable carrier. It has been surprisingly discovered that such compositions comprising extracts of *Carum carvi* seed extract and *Rosmarinus officinalis* extract provide unexpectedly good anti-inflammatory properties. When combined, the extracts exhibit a synergistic anti-inflammatory effect. It has been demonstrated all tested combinations of *Carum carvi* seed extract and *Rosmarinus officinalis* are synergistic (having a >1-fold increase from additive levels), and even a 1.5- or 2-fold increase in some combinations. In fact, it has been demonstrated that there are instances where one or both of the extracts may provide no significant anti-inflammatory result for a given marker, yet exhibit strong anti-inflammation properties when the two extracts are combined.

### Carum carvi Seed Extract

*Carum carvi,* commonly known as caraway, is a biennial plant native to parts of Asia, Europe and Africa. As used herein *"Carum carvi* seed extract" refers to an extract of the seed from the plant. In the present invention, the *Carum carvi* seed extract comprises a polar extract, wherein the polar extract was extracted using one or more of C₁-C₈ alcohols, C₁-C₈ polyols, C₁-C₈ glycols and combinations of two or more thereof. Any suitable manner of preparing the extracts of *Carum carvi* seed extract for use in accordance with the present invention may be used. Suitable extracts may be obtained using conventional methods including, but not limited to, direct extraction from the biomass by grinding, macerating, pressing, squeezing, mashing, centrifuging, and/or processes such as cold percolation, agitation/distillation, microwave assisted extraction, sonication, supercritical/subcritical CO₂ compressed gas extraction with or without polarity modifier, pressurized solvent extraction, accelerated solvent extraction, surfactant assisted pressurized hot water extraction, oil extraction, membrane extraction, Soxhlet extraction, the gold finger distillation/extraction and/or processes disclosed, for example, in US Pat. Nos. 7,442,391, 7,473,435, and 7,537,791 to Integrated Botanical Technologies, LLC, or by other methods such as solvent extraction, and the like. In particular, an extract in accordance with the present invention may be a solvent-based extraction made by grinding or macerating plant material in a solvent, typically an organic solvent such as an alcohol, acetone, liquid carbon dioxide with or without polarity modifier, hexane, or chloroform. The resulting extract comprises mainly non-polar compounds. The plant biomass may be separated entirely from the extraction, and not used after extraction.

Any of a variety of solvents including polar solvents, non-polar solvents, or combinations of two or more thereof may be used in methods of comprising solvent extraction.

Suitable polar solvents include polar inorganic solvents such as water and the like, polar organic solvents such as alcohols and corresponding organic acids, for example C₁-C₈ alcohols including methanol, ethanol, propanol, butanol, and the like and organic acids, including acetic acid, formic acid, propanoic acid, and the like, polyols and glycols, including C₁-C₈ polyols/glycols and the like, and combinations of two or more thereof. Suitable non-polar solvents include non-polar organic solvents such as alkanes, including C₁-C₈ alkanes, cycloalkanes, including C₁-C₈ alkanes, alkyl ethers, including C₁-C₈ alkyl ethers, Petroleum ethers, ketones, including C₁-C₈ ketones, methylene chloride, ethyl acetate, xylene, toluene, chloroform, vegetable oil, mineral oil and the like. In another embodiment extraction may be obtained by non-polar solvents described above or supercritical fluid extraction with or without a polar modifier such as C₁-C₈ alcohols, water, C₁-C₈ polyols/glycols or C₁-C₈ organic acids.

In one or more embodiments, the extract is a polar extract. Polar extract means the extract is produced by subjecting the plant or parts of the plant to a polar solvent. In a certain embodiment, the extract is prepared by pulverizing the seeds of *Carum carvi* and extracting using a polar solvent having a dielectric constant value of between 1 and 100 at 20°C, specifically a dielectric constant of a value between 4 and 60 at 20°C, more specifically a dielectric constant of a value between 4 and 50 at 20°C, and even more specifically a dielectric constant of a value between 4 and 40 at 20°C. Examples of suitable polar solvents include C₁-C₈ alcohols, C₁-C₈ polyols/glycols, C₁-C₈ organic acids, water and combinations of two or more thereof having a dielectric constant value of between 1 and 100, specifically between 4 and 60, and more specifically between 5 and 40 at 20°C, including, but not limited to, those solvents and combinations of solvents having the desired dielectric constant value as disclosed in "Dielectric Constants of Some Organic Solvent-Water Mixtures at Various Temperatures," Akerlof, Gosta; JACS, Vol. 54, No. 11 (Nov. 1932), pp. 4125-4139. The polar extract is extracted using one or more C₁-C₈ alcohols, C₁-Cs polyols, C₁-C₈ glycols, and combinations of two or more thereof. In one or more embodiments, the extract is extracted using one or more C₁-C₄ alcohols, C₁-C₄ polyols, and/or C₁-C₄ glycols. In some embodiments, the extract is prepared using a solvent comprising methanol, ethanol, or a combination thereof with or without presence of water. In one or more embodiments, the extract may be further refined by charcoal (also referred to as active carbon) treatment. In one or more embodiments, the extract is an aqueous alcohol extract (*i.e.,* a mixture of alcohol in water). In some embodiments, the aqueous alcohol extract comprises between about 5% and 10% alcohol in water.

In some embodiments, the extract of the invention is an extract prepared by pulverizing the *Carum carvi* seeds and extracting using a solvent having a dielectric constant of a value between about 1 and about 80 at 20°C, specifically a dielectric constant of a value between about 2 and about 60 at 20°C, more specifically a dielectric constant of a value between about 2 and about 40 at 20°C, and even more specifically a dielectric constant of a value between about 2 and 35 at 20°C.

In some embodiments, the composition is essentially free from extracts of other non-seed parts of *Carum carvi* plant. In further embodiments, the composition is free from extracts of other non-seed parts of *Carum carvi* plant. In certain embodiments, the composition may comprise extracts from cell cultures of plants of *Carum carvi.* In one or more embodiments, the *Carum carvi* seed extract is substantially free of nonpolar extracts. In further embodiments, the *Carum carvi* seed extract is free of nonpolar extracts. In some embodiments, the *Carum carvi* seed extract and *Rosmarinus officinalis* extract are both substantially free of nonpolar extracts. In further embodiments, the *Carum carvi* seed extract and *Rosmarinus officinalis* extract are both free of nonpolar extracts. In one or more embodiments, the *Carum carvi* seed extract is substantially free of essential oils. In one or more embodiments, the *Carum carvi* seed extract is free of essential oils. In further embodiments, the *Carum carvi* seed extract and *Rosmarinus officinalis* extract are both substantially free of essential oils. In yet further embodiments, the *Carum carvi* seed extract and *Rosmarinus officinalis* extract are both free of essential oils. In some embodiments, the *Carum carvi* seed extract and *Rosmarinus officinalis* extract are both substantially free of volatile components. In some embodiments, the *Carum carvi* seed extract and *Rosmarinus officinalis* extract are both free of volatile components.

Any suitable amount of *Carum carvi* seed extract may be used in the topical compositions of the present invention. In one or more embodiments, the compositions comprise from greater than zero to about 20% *Carum carvi* seed extract. In some embodiments, the compositions comprise from about 0.0001 to about 20%, from about 0.001 to about 10%, from about 0.01 to about 5%, from about 0.1 to about 5%, or from about 0.2 to about 2% *Carum carvi* seed extract. In one or more, the compositions comprise from greater than zero to about 1%, from about 0.0001 to about 1%, from about 0.001 to about 1%, or from about 0.01 to about 1% *Carum carvi* seed extract. In some embodiments, the compositions comprise from about 1 to about 5%, specifically from about 2 to about 5% *Carum carvi* seed extract.

### Rosmarinus officinalis Extract

As used herein, the term *"Rosmarinus officinalis* extract" refers to an extract from any of the aerial parts of the *Rosmarinus officinalis* plant for example, one or more of the stem or leaves. In other embodiments, the composition is essentially free from extracts of other non-stem and/or non-leaf parts of *Rosmarinus officinalis.*

In the present invention, the *Rosmarinus officinalis* leaf extract comprises a polar extract, wherein the polar extract was extracted using one or more of C₁-C₈ alcohols, C₁-C₈ polyols, C₁-C₈ glycols and combinations of two or more thereof. Any suitable manner of preparing the extracts of *Rosmarinus officinalis* for use in accordance with the present invention may be used. Suitable extracts may be obtained using conventional methods including, but not limited to, direct extraction from the biomass by grinding, macerating, pressing, squeezing, mashing, centrifuging, and/or processes such as cold percolation, agitation/distillation, microwave assisted extraction, sonication, supercritical/subcritical CO₂ compressed gas extraction with or without polarity modifier, pressurized solvent extraction, accelerated solvent extraction, surfactant assisted pressurized hot water extraction, oil extraction, membrane extraction, Soxhlet extraction, the gold finger distillation/extraction and/or processes disclosed, for example, in US Pat. Nos. 7,442,391, 7,473,435, and 7,537,791 to Integrated Botanical Technologies, LLC, or by other methods such as solvent extraction, and the like. In particular, an extract in accordance with the present invention may be a solvent-based extraction made by grinding or macerating plant material in a solvent, typically an organic solvent such as an alcohol, acetone, liquid carbon dioxide with or without polarity modifier, hexane, or chloroform. The resulting extract comprised mainly non-polar compounds. The plant biomass may be separated entirely from the extraction, and is not used after extraction.

Any of a variety of solvents including polar solvents, non-polar solvents, or combinations of two or more thereof may be used in methods of comprising solvent extraction. Suitable polar solvents include polar inorganic solvents such as water and the like, polar organic solvents such as alcohols and corresponding organic acids, for example C₁-C₈ alcohols including methanol, ethanol, propanol, butanol, and the like and organic acids, including acetic acid, formic acid, propanoic acid, and the like, polyols and glycols, including C₁-C₈ polyols/glycols and the like, and combinations of two or more thereof. Suitable non-polar solvents include non-polar organic solvents such as alkanes, including C₁-C₈ alkanes, cycloalkanes, including C₁-C₈ alkanes, alkyl ethers, including C₁-C₈ alkyl ethers, Petroleum ethers, ketones, including C₁-C₈ ketones, methylene chloride, ethyl acetate, xylene, toluene, chloroform, vegetable oil, mineral oil and the like. In another embodiment extraction may be obtained by non-polar solvents described above or supercritical fluid extraction with or without a polar modifier such as C₁-C₈ alcohols, water, C₁-C₈ polyols/glycols or C₁-C₈ organic acids.

In one or more embodiments, the extract is a polar extract. Polar extract means the extract is produced by subjecting the plant or parts of the plant to a polar solvent. In a certain embodiment, the extract is prepared by pulverizing the leaves of *Rosmarinus officinalis* and extracting using a polar solvent having a dielectric constant value of between 1 and 100 at 20°C, specifically a dielectric constant of a value between 4 and 60 at 20°C, more specifically a dielectric constant of a value between 4 and 50 at 20°C, and even more specifically a dielectric constant of a value between 4 and 40 at 20°C. Examples of suitable polar solvents include C₁-C₈ alcohols, C₁-C₈ polyols/glycols, C₁-C₈ organic acids, water and combinations of two or more thereof having a dielectric constant value of between 1 and 100, specifically between 4 and 60, and more specifically between 5 and 40 at 20°C, including, but not limited to, those solvents and combinations of solvents having the desired dielectric constant value as disclosed in "Dielectric Constants of Some Organic Solvent-Water Mixtures at Various Temperatures," Akerlof, Gosta; JACS, Vol. 54, No. 11 (Nov. 1932), pp. 4125-4139. The polar extract is extracted using one or more C₁-C₈ alcohols, C₁-Cs polyols, C₁-C₈ glycols, and combinations of two or more thereof. In some embodiments, the extract is extracted using one or more C₁-C₄ alcohols, C₁-C₄ polyols, and/or C₁-C₄ glycols. In one or more embodiments, the extract is prepared using a solvent comprising methanol, ethanol, or a combination thereof with or without presence of water. In some embodiments, the extract may be further refined by charcoal (also referred to as active carbon) treatment. In one or more embodiments, the extract is an aqueous alcohol extract (*i.e.,* a mixture of alcohol in water). In some embodiments, the aqueous alcohol extract comprises between about 5% and 10% alcohol in water.

In one or more embodiments, the extract of the invention is an extract prepared by pulverizing the *Rosmarinus officinalis* leaves and extracting using a solvent having a dielectric constant of a value between about 1 and about 80 at 20°C, specifically a dielectric constant of a value between about 2 and about 60 at 20°C, more specifically a dielectric constant of a value between about 2 and about 40 at 20°C, and even more specifically a dielectric constant of a value between about 2 and 35 at 20°C.

In certain embodiments, the composition may comprise extracts from cell cultures of aerial parts of the *Rosmarinus officinalis* plant. In one or more embodiments, the *Rosmarinus officinalis* extract is substantially free of nonpolar extracts. In further embodiments, the *Carum carvi* seed extract and *Rosmarinus officinalis* extract are both substantially free of nonpolar extracts. In one or more embodiments, the *Rosmarinus officinalis* extract is substantially free of essential oils. In one or more embodiments, the *Rosmarinus officinalis* extract is free of essential oils. In further embodiments, the *Carum carvi* seed extract and *Rosmarinus officinalis* extract are both substantially free of essential oils. In further embodiments, the *Carum carvi* seed extract and *Rosmarinus officinalis* extract are both free of essential oils. In some embodiments, the *Carum carvi* seed extract and *Rosmarinus officinalis* extract are both substantially free of volatile components.

Any suitable amount of *Rosmarinus officinalis* extract may be used in the topical compositions of the present invention. In some embodiments, the compositions comprise from greater than zero to about 20% *Rosmarinus officinalis* extract. In one or more embodiments, the compositions comprise from about 0.0001 to about 20%, from about 0.001 to about 10%, from about 0.01 to about 5%, from about 0.1 to about 5%, or from about 0.2 to about 2% *Rosmarinus officinalis* extract. In some embodiments, the compositions comprise from greater than zero to about 1%, from about 0.0001 to about 1%, from about 0.001 to about 1%, or from about 0.01 to about 1% *Rosmarinus officinalis* extract. In one or more embodiments, the compositions comprise from about 1 to about 5%, specifically from about 2 to about 5% *Rosmarinus officinalis* extract.

Any suitable amount of extracts of *Carum carvi* seed and *Rosmarinus officinalis* may be used in the compositions of the present invention. In one or more embodiments, the compositions comprise a safe and effective amounts of extracts of *Carum carvi* seed and *Rosmarinus officinalis* extracts. In particular, the amounts of the extracts of *Carum carvi* seed and *Rosmarinus officinalis* to be used may be selected to achieve the desired treatment of a given inflammatory condition. In some embodiments, the compositions comprise from about 0.0001 to about 20%, from about 0.001 to about 10%, from about 0.01 to about 5%, from about 0.1 to about 5%, or from about 0.2 to about 2% of *Carum carvi* seed and *Rosmarinus officinalis* extracts combined. In one or more embodiments, the compositions comprise from greater than zero to about 1%, from about 0.0001 to about 1%, from about 0.001 to about 1%, or from about 0.01 to about 1% *Carum carvi* seed and *Rosmarinus officinalis* extracts combined. In some embodiments, the compositions comprise from about 1 to about 5%, specifically from about 2 to about 5% *Carum carvi* seed and *Rosmarinus officinalis* extracts combined. The extracts of *Carum carvi* seed and *Rosmarinus officinalis* extracts are present in the composition in amounts which may vary depending on the end point relevant to the desired benefit. For example, in some embodiments, in particular those in which binding of the CCR2 receptor is desired, the weight ratio of *Rosmarinus officinalis* extract to *Carum carvi* seed extract is from about 1:9 to about 1:70, more specifically from about 1:17 to about 1:35, and more specifically about 1:35. In some embodiments, in particular those in which reduction of Interleukin 6 and 8 (IL-6, IL-8) release is desired, the weight ratio of *Rosmarinus officinalis* extract to *Carum carvi* seed extract is about 1:2 to about 1:100, more specifically from about 1:10 to about 1:100, and more specifically about 1:100.

### Carrier

Any suitable carrier may be used in the compositions. In some embodiments, the carrier is a cosmetically-acceptable carrier. As will be recognized by those of skill in the art, cosmetically acceptable carriers comprise carriers that are suitable for use in contact with the body, in particular the skin, without undue toxicity, incompatibility, instability, irritation, allergic response, and the like. A safe and effective amount of carrier is from about 50, 55, 60, 65, 70, 75, 80, 85, 90, 95, or 98% to about 85, 90, 95, 98, 99, 99.1, 99.5 or 99.9% by weight of the composition.

The carrier can be in a wide variety of forms. For example, carriers in the form of emulsions, including, but not limited to, oil-in-water, water-in-oil, water-in-oil-in-water, and oil-in-water-in-silicone emulsions, are useful herein. These emulsions can cover a broad range of viscosities, *e.g.,* from about 100 cps to about 200,000 cps using a Brookfield RVT viscometer.

Examples of suitable cosmetically-acceptable carriers include cosmetically acceptable solvents and materials for cosmetic solutions, suspensions, lotions, creams, serums, essences, gels, toners, sticks, sprays, ointments, liquid washes and soap bars, shampoos, hair conditioners, pastes, foams, mousses, powders, shaving creams, wipes, patches, strips, powered patches, micro-needle patches, bandages, hydrogels, film-forming products, facial and skin masks, make-up, liquid drops, and the like. These product types may contain several types of cosmetically- acceptable carriers including, but not limited to solutions, suspensions, emulsions such as micro-emulsions and nano-emulsions, gels, solids, liposomes, other encapsulation technologies and the like.

The following are non-limiting examples of carriers. Other carriers can be formulated by those of ordinary skill in the art. In one embodiment, the carrier contains water. In a further embodiment, the carrier may also contain one or more aqueous or organic solvents. Examples of organic solvents include, but are not limited to: dimethyl isosorbide; isopropyl myristate; surfactants of cationic, anionic and nonionic nature; vegetable oils; mineral oils; waxes; gums; synthetic and natural gelling agents; alkanols; glycols; and polyols. Examples of glycols include, but are not limited to, glycerin, propylene glycol, butylene glycol, pentalene glycol, hexylene glycol, polyethylene glycol, polypropylene glycol, diethylene glycol, triethylene glycol, capryl glycol, glycerol, butanediol and hexanetriol, and copolymers or mixtures thereof. Examples of alkanols include, but are not limited to, those having from about 2 carbon atoms to about 12 carbon atoms (e.g., from about 2 carbon atoms to about 4 carbon atoms), such as isopropanol and ethanol. Examples of polyols include, but are not limited to, those having from about 2 carbon atoms to about 15 carbon atoms (*e.g.,* from about 2 carbon atoms to about 10 carbon atoms) such as propylene glycol. The organic solvents may be present in the carrier in an amount, based upon the total weight of the carrier, of from about 1 percent to about 99.99 percent (*e.g.,* from about 20 percent to about 50 percent). Water may be present in the carrier (prior to use) in an amount, based upon the total weight of the carrier, of from about 5 percent to about 95 percent (e.g., from about 50 percent to about 90 percent). Solutions may contain any suitable amounts of solvent, including from about 40 to about 99.99%. Some solutions contain from about 50 to about 99.9%, from about 60 to about 99%, from about 70 to about 99%, from about 80 to about 99%, or from about 90 to 99% of solvent.

A lotion can be made from such a solution. Lotions typically contain at least one emollient in addition to a solvent. Lotions may comprise from about 1% to about 20% (e.g., from about 5% to about 10%) of an emollient(s) and from about 50% to about 90% (e.g., from about 60% to about 80%) of water.

Another type of product that may be formulated from a solution is a cream. A cream typically contains from about 5% to about 50% (*e.g.,* from about 10% to about 20%) of an emollient(s) and from about 45% to about 85% (*e.g.,* from about 50% to about 75%) of water.

Yet another type of product that may be formulated from a solution is an ointment. An ointment may contain a simple base of animal, vegetable, or synthetic oils or semi-solid 10 hydrocarbons. An ointment may contain from about 2% to about 10% of an emollient(s) plus from about 0.1% to about 2% of a thickening agent(s).

The compositions useful in the present invention can also be formulated as emulsions. If the carrier is an emulsion, from about 1% to about 10% (*e.g.,* from about 2% to about 5%) of the carrier contains an emulsifier(s). Emulsifiers may be nonionic, anionic or cationic.

Lotions and creams can be formulated as emulsions. Typically such lotions contain from 0.5% to about 5% of an emulsifier(s), while such creams would typically contain from about 1% to about 20% (e.g., from about 5% to about 10%) of an emollient(s); from about 20% to about 80% (e.g., from 30% to about 70%) of water; and from about 1% to about 10% (e.g., from about 2% to about 5%) of an emulsifier(s).

Single emulsion skin care preparations, such as lotions and creams, of the oil-in-water type, and water-in-oil type are well-known in the art and are useful in the subject invention. Multiphase emulsion compositions, such as the water-in-oil-in-water type or the oil-in-water-in-oil type, are also useful in the subject invention. In general, such single or multiphase emulsions contain water, emollients, and emulsifiers as essential ingredients.

The compositions of this invention can also be formulated as a gel (*e.g.,* an aqueous, alcohol, alcohol/water, or oil gel using a suitable gelling agent(s)). Suitable gelling agents for aqueous and/or alcoholic gels include, but are not limited to, natural gums, acrylic acid and acrylate polymers, and copolymers, and cellulose derivatives (*e.g.,* hydroxymethyl cellulose and hydroxypropyl cellulose). Suitable gelling agents for oils (such as mineral oil) include, but are not limited to, hydrogenated butylene/ethylene/styrene copolymer and hydrogenated ethylene/propylene/styrene copolymer. Such gels typically contains between about 0.1% and 5%, by weight, of such gelling agents.

The compositions of the present invention can also be formulated into a solid formulation (*e.g.,* a wax-based stick, soap bar composition, powder, or wipe). The composition of the present invention can also be combined with a solid, semi-solid, or dissolvable substrate (*e.g.,* a wipe, mask, pad, glove, or strip).

### Other Additives

The compositions of the present invention may further comprise any of a variety of additional cosmetically active agents. Examples of suitable additional active agents include: skin lightening agents, darkening agents, additional anti-aging agents, tropoelastin promoters, collagen promoters, anti-acne agents, shine control agents, antimicrobial agents such as anti-yeast agents, anti-fungal, and anti-bacterial agents, anti-inflammatory agents, anti-parasite agents, external analgesics, sunscreens, photoprotectors, antioxidants, keratolytic agents, detergents/surfactants, moisturizers, nutrients, vitamins, energy enhancers, anti-perspiration agents, astringents, deodorants, hair removers, hair growth enhancing agents, hair growth delaying agents, firming agents, hydration boosters, efficacy boosters, anti-callous agents, agents for skin conditioning, anti-cellulite agents, odor-control agents such as odor masking or pH changing agents, and the like.

Examples of various suitable additional cosmetically acceptable actives include hydroxy acids; benzoyl peroxide; D-panthenol; UV filters such as but not limited to avobenzone (Parsol 1789), bisdisulizole disodium (Neo Heliopan AP), diethylamino hydroxybenzoyl hexyl benzoate (Uvinul A Plus), ecamsule (Mexoryl SX), methyl anthranilate, 4-aminobenzoic acid (PABA), cinoxate, ethylhexyl triazone (Uvinul T 150), homosalate, 4-methylbenzylidene camphor (Parsol 5000), octyl methoxycinnamate (Octinoxate), octyl salicylate (Octisalate), padimate O (Escalol 507), phenylbenzimidazole sulfonic acid (Ensulizole), polysilicone-15 (Parsol SLX), trolamine salicylate, Bemotrizinol (Tinosorb S), benzophenones 1-12, dioxybenzone, drometrizole trisiloxane (Mexoryl XL), iscotrizinol (Uvasorb HEB), octocrylene, oxybenzone (Eusolex 4360), sulisobenzone, bisoctrizole (Tinosorb M), titanium dioxide, zinc oxide; carotenoids; free radical scavengers; spin traps; retinoids and retinoid precursors such as retinol, retinoic acid and retinyl palmitate; ceramides; polyunsaturated fatty acids; essential fatty acids; enzymes; enzyme inhibitors; minerals; hormones such as estrogens; steroids such as hydrocortisone; 2-dimethylaminoethanol; copper salts such as copper chloride; peptides containing copper such as Cu:Gly-His-Lys, coenzyme Q10; amino acids such a proline; vitamins; lactobionic acid; acetyl-coenzyme A; niacin; riboflavin; thiamin; ribose; electron transporters such as NADH and FADH2; and other botanical extracts such as oat, aloe vera, Feverfew, Soy, Shiitake mushroom extracts, and derivatives and mixtures thereof.

If present, any additional cosmetically active agent may be present in a composition in any suitable amount, for example, in an amount of from about 0.0001% to about 20% by weight of the composition, e.g., about 0.001% to about 10% such as about 0.01% to about 5%. In some embodiments. in an amount of 0.1% to 5% and in other embodiments from 1% to 2%.

Compositions of the present invention may include a cosmetically effective amount of one or more additional anti-inflammatory compounds. Examples of suitable anti-inflammatory agents include substituted resorcinols, (E)-3-(4-methylphenylsulfonyl)-2-propenenitrile (such as "Bay 11-7082," commercially available from Sigma-Aldrich of St. Louis, Missouri), tetrahydrocurcuminoids (such as Tetrahydrocurcuminoid CG, available from Sabinsa Corporation of Piscataway, NJ), extracts and materials derived from the following: *Phellodendron amurense* Cortex Extract (PCE), Non-Denatured Soy (*Glycine max*), Feverfew (*Tanacetum parthenium*), Ginger (*Zingiber officinale*), Ginkgo (*Ginkgo biloba*), Madecassoside (*Centella asiatica* extract ingredient), Cotinus (*Cotinus coggygria*), Butterbur Extract (*Petasites hybridus*), Goji Berry (*Lycium barbarum*), Milk Thistle Extract (*Silybum marianum*)*,* Honeysuckle (*Lonicera japonica*), Basalm of Peru (*Myroxylon pereirae*), Sage (*Salvia officinalis*), Cranberry Extract (*Vaccinium oxycoccos*), Amaranth Oil (*Amaranthus cruentus*), Pomegranate (*Punica granatum*), Yerbe Mate (*Ilex paraguariensis* Leaf Extract), White Lily Flower Extract (*Lilium candidum*), Olive Leaf Extract (*Olea europaea*), Phloretin (apple extract), Oat Flour (*Aveena sativa*), Lifenol (Hops: *Humulus lupulus*) Extract, Bugrane P (*Ononis spinosa*), Licochalcone (Licorice: *Glycyrrhiza inflate* extract ingredient), Symrelief (Bisabolol and Ginger extract), combinations of two or more thereof, and the like.

In one embodiment, the anti-inflammatory agent is a resorcinol. Particularly suitable substituted resorcinols include 4-hexyl resorcinol and 4-octylresorcinol, particularly 4-hexyl resorcinol. 4-Hexyl resorcinol is commercially available as "SYNOVEA HR" from Sytheon of Lincoln Park, NJ. 4-Octylresorcinol is commercially available from City Chemical LLC of West Haven, Connecticut.

By "extracts of feverfew," it is meant extracts of the plant *"Tanacetum parthenium,"* such as may be produced according to the details set for the in US Patent No. 7,537,791, entitled "PARTHENOLIDE FREE BIOACTIVE INGREDIENTS FROM FEVERFEW (TANACETUM PARTHENIUM) AND PROCESSES FOR THEIR PRODUCTION." One particularly suitable feverfew extract is commercially available as about 20% active feverfew, from Integrated Botanical Technologies of Ossining, NY.

A variety of other materials may also be present in the compositions of the present invention. In one or more embodiments, the composition comprises one or more topical ingredients selected from the group consisting of: surfactants, chelating agents, emollients, humectants, conditioners, preservatives, opacifiers, fragrances and the like.

What is meant by an emollient is a compound that helps to maintain the soft, smooth, and pliable appearance of the skin (*e.g.,* by remaining on the skin surface or in the stratum corneum to act as a lubricant). Examples of suitable emollients include those found in Chapter 35, pages 399-415 (Skin Feel Agents, by G Zocchi) in Handbook of Cosmetic Science and Technology (edited by A. Barel, M. Paye and H. Maibach, Published in 2001 by Marcel Dekker, Inc New York, NY), and include, but are not limited to, petrolatum, hexyldecyl stearate and plant, nut, and vegetable oils such as macadamia nut oil, rice bran oil, grape seed oil, palm oil, prim rose oil, hydrogenates peanut oil, and avocado oil.

What is meant by a humectant is a compound intended to increase the water content of the top layers of skin (*e.g.,* hygroscopic compounds). Examples of suitable humectants include those found in Chapter 35, pages 399-415 (Skin Feel Agents, by G Zocchi) in Handbook of Cosmetic Science and Technology (edited by A. Barel, M. Paye and H. Maibach, Published in 2001 by Marcel Dekker, Inc New York, NY) and include, but are not limited to, glycerin, sorbitol or trehalose (*e.g*., α,α- trehalose, β,β-trehalose, α,β-trehalose) or a salt or ester thereof (*e.g.,* trehalose 6-phosphate).

What is meant by a surfactant is a surface-active agent intended to cleanse or emulsify. Examples of suitable surfactants include those found in Chapter 37, pages 431-450 (Classification of surfactants, by L. Oldenhove de Guertechin) in Handbook of Cosmetic Science and Technology (edited by A. Barel, M. Paye and H. Maibach, Published in 2001 by Marcel Dekker, Inc., New York, NY) and include, but are not limited to anionic surfactants such as sulfates, cationic surfactants such as betaines, amphoteric surfactants such as sodium coco glycinate, nonionic surfactants such as alkyl polyglucosides.

Examples of suitable chelating agents include those which are capable of protecting and preserving the compositions of this invention. In one or more embodiments, the chelating agent is ethylenediaminetetraacetic acid ("EDTA"), and more specifically is tetrasodium EDTA, available commercially from Dow Chemical Company of Midland, Michigan under the trade name, "Versene 100XL."

Suitable preservatives include, for example, parabens, quaternary ammonium species, phenoxyethanol, benzoates, DMDM hydantoin, organic acids and are present in the composition in an amount, based upon the total weight of the composition, from about 0 to about 1 percent or from about 0.05 percent to about 0.5 percent.

Any of a variety of conditioners which impart additional attributes, such as gloss to the hair, are suitable for use in this invention. Examples include, but are not limited to, volatile silicone conditioning agent having an atmospheric pressure boiling point less than about 220°C. Examples of suitable volatile silicones nonexclusively include polydimethylsiloxane, polydimethylcyclosiloxane, hexamethyldisiloxane, cyclomethicone fluids such as polydimethylcyclosiloxane available commercially from Dow Corning Corporation of Midland, Michigan under the tradename, "DC-345" and mixtures thereof, and specifically include cyclomethicone fluids. Other suitable conditioners include cationic polymers, including polyquarterniums, cationic guar, and the like.

Any of a variety of commercially available pearlescent or opacifying agents are suitable for use in the composition. Examples of suitable pearlescent or opacifying agents include, but are not limited to, mono or diesters of (a) fatty acids having from about 16 to about 22 carbon atoms and (b) either ethylene or propylene glycol; mono or diesters of (a) fatty acids having from about 16 to about 22 carbon atoms (b) a polyalkylene glycol of the formula: HO-(JO)a-H, wherein J is an alkylene group having from about 2 to about 3 carbon atoms; and a is 2 or 3; fatty alcohols containing from about 16 to about 22 carbon atoms; fatty esters of the formula: KCOOCH₂L, wherein K and L independently contain from about 15 to about 21 carbon atoms; inorganic solids insoluble in the shampoo composition, and mixtures thereof.

Any fragrance compositions suitable for use on skin may be used in the composition according to the present invention.

### Methods and Product Form

The present invention further comprises a method of reducing inflammation by administering to a patient in need thereof a therapeutically effective dose of a composition comprising *Carum carvi* seed extract and *Rosmarinus officinalis* extract. In some embodiments, the inflammation is CCR2 receptor-mediated inflammation.

The composition may be applied topically. Such topical application may be to any skin in need of treatment on the body, for example skin of the face, lips, neck, chest, back, buttocks, arms, axilla, and/or legs. The composition may also be administered to a mucous membrane (*i.e.,* in the oral cavity). In some embodiments, the extracts are polar extracts of *Carum carvi* seed extract and *Rosmarinus officinalis* extract.

In some embodiments, the present invention is in the form of a substrate comprising a composition of the present invention. Any suitable substrate may be used. Examples of suitable substrates and substrate materials are disclosed, for example, in US7452547 and US2009/0241242. In some embodiments, the composition is in the form of a tablet, pill, or capsule. In one or more embodiments, the composition is in the form of a solution, suspension, emulsion, lotion, cream, serum, gel, stick, spray, ointment, liquid wash, soap bar, shampoo, hair conditioner, paste, foam, powder, mousse, shaving cream, hydrogel, or film-forming product.

Any suitable method of applying the composition to the skin in need may be used. For example, the composition may be applied directly from a package to the skin in need, by hand to the skin in need, or may be transferred from a substrate such as a wipe or mask, or a combination of two or more thereof. In other embodiments, the composition may be applied via a dropper, tube, roller, spray, and patch or added to a bath or otherwise to water to be applied to the skin, and the like. The composition may be applied in a variety of manners or forms, including, without limitation, as a leave-on cream, mask, and /or serum.

The presently disclosed embodiments are to be considered in all respects as illustrative and not restrictive, the scope of the invention being indicated by the appended claims, and not limited to the foregoing description. It will be appreciated that in the claims, the term "comprises/comprising" does not exclude the presence of other elements or steps. In addition, singular references do not exclude a plurality. The terms "a", "an", "first", "second", etc., do not preclude a plurality.

All percentages, parts and ratios are based upon the total weight of the composition of the present invention, unless otherwise specified. All such weights as they pertain to the listed ingredients are based on the level of the particular ingredient described and, therefore, do not include carriers or by-products that may be included in commercially available materials, unless otherwise specified.

### EXAMPLES

The following test methods and materials were used in the Examples.

### Assay 1: C-C Chemokine Receptor Type 2 (CCR2) Antagonism Assay

THP1 cells (American Type Culture Collection, Manassas, VA, catalog name THP-1 (ATCC^{®} TIB-202^{™}) were plated in 384 well polystyrene poly-d-lysine flat bottom cell culture microplate with lid and black clear bottom, sold under the tradename CELLCOAT^{™} (Greiner Bio-One, Monroe, NC, catalog number 781946), at a density of 15k cells/well in 40ul assay buffer (HBSS/20mM HEPES/0.001% Tween^{®} 20) using a Combi reagent dispenser, sold under the trade name Multidrop^{™} (ThermoFisher Scientific, Waltham, MA) by mixing cells at a density of 7.5E5 cells/mL in buffer 1:1 with buffer containing the calcium dye reagents using a calcium assay kit sold under the tradename Screen Quest^{™} Fluo-8 No Wash Calcium Assay Kit (AAT Bioquest, Sunnydale, CA, catalog number 36316). Plates were spun down at 800 rpm for 2 min with no brakes. Cells were then incubated at 37 °C in the incubator for 45 min, and then placed at room temperature for 15 min. CCR2 antagonism was assessed using the FLIPR Penta High Throughput Cellular Screening System (Molecular Devices, San Jose, CA) by measuring calcium flux caused by activation of the CCR2 receptor by its ligand MCP1 (Peprotech, Inc., Rocky Hill, NJ, catalog number 300-04) at an EC80 dose of 200 nM. Using a 2-addition protocol with Ex/Em 490/525 settings, baseline reads were taken (5x1 sec), subsequently 10µL of buffer containing extracts was added to the cells, and calcium flux was measured (60x1 sec, followed by 40x3 sec reads). Next, 10 µl agonist (MCP1 at 200nM final concentration) was added and calcium flux was measured (60x1 sec, followed by 40x3 sec reads).

For analysis, the first phase in which extracts were added is considered the agonist phase, where it is determined whether the extracts have any agonistic effect on calcium flux. The flux observed in control wells treated with anhydrous DMSO (Sigma-Aldrich, St. Louis, MO) is set to 0% agonism, and the flux observed in control wells treated with agonist MCP1 is set to 100% agonism. In the second phase in which the EC80 dose of MCP1 is added to every well in the plate, the antagonism of the extracts is determined. The flux observed in wells treated with DMSO (from first phase) is set to 0% antagonism, and the flux observed in wells treated with control antagonism (from first phase) is set to 100% antagonism. Flux in both agonist and antagonist phase is the difference between the highest calcium levels observed in that phase and the initial baseline.

For the synergy assay, 2 plates were used, with two sets of synergy experiments on each of those plates, and each condition in triplicate per experiment.

### Assay 2: Cytokine release assay

Normal Human Epidermal Keratinocytes, P2, lot EP-BA, were sourced (Sterlab, Vallauris, France) and seeded in 96 well plate in reconstituted keratinocyte growth media sold under the trade name (KGM^{™} Gold Keratinocyte Growth medium Bulletkit^{™}, Lonza, Verviers, Belgium) at 30000 cells/well. The next day media was replaced by keratinocyte growth media containing the plant extracts and heat-inactivated *P. acnes* (10⁹ CFU, strain ID ATCC^{®} 6919) sourced from LGC Standards, Molsheim, France. The following day conditioned media was collected to measure IL6, IL8 and TNFa release using Bio-Plex suspension array system, sold under the trade name BIOPLEX 200, Bio-Rad Laboratories, Inc., Marnes-la-Coquette, France, with respective standard reference material kits, and cell viability was assessed using an MTT test. Every condition had 4 replicates. Tested extracts included Rosemary leaf extract at 0.5, 5 and 25 µg/mL final concentration, Caraway seed extract at 50 µg/mL final concentration, and the combinations of these two extracts. The effects of *P. acnes* alone were normalized to 100% for the cytokine release assays. Relative inhibition by the co-treatments was calculated by following formula: 1 - {(observed - DMSO treated)/(P. Acnes - DMSO treated)}.

### Rosemary Leaf Extract

Rosemary leaf (*Rosmarinus officinalis*) extract was obtained from Ungerer & Company of New Jersey, USA. The rosemary leaf extract was prepared with organic/aqueous medium comprised of water and alcohol mixture. A typical preparation was done mixing biomass with a mixture of water / denatured ethyl alcohol (2:8, respectively) at room temperature for 24h, filtered and filtrate dried under reduced pressure to obtain sold material - the extract. The extract was suspended in anhydrous DMSO (Sigma-Aldrich) at 100mg/mL stock solution. The stock solution was then serially diluted to conduct efficacy testing according to the assay protocols described above.

### Caraway Seed Extract

Caraway seed (*Carum carvi*) extract was obtained from Ungerer & Company of New Jersey, USA. The Caraway seed extract was prepared with organic/aqueous medium comprised of water and alcohol mixture. A typical preparation was done mixing biomass with a mixture of water / ethyl alcohol (2:8, respectively) at room temperature for 24h, filtered and filtrate dried under reduced pressure to obtain sold material - the extract. The extract was suspended in anhydrous DMSO (Sigma-Aldrich) at 100mg/mL stock solution. The stock solution was then serially diluted to conduct efficacy testing according to the assay protocols described above.

### EXAMPLE 1: Inhibition of CCR2 Receptors

Rosemary leaf extract and caraway seed extract were tested alone and in combinations of varying concentrations and evaluated for inhibition of the CCR2 receptor according to Assay 1 above. The CCR2 receptor is a chemokine receptor which is associated with a wide variety of inflammatory diseases/conditions. Inhibition of the receptor is thus associated with reduction in inflammation. The results of the experiment are shown in Table 1 below.

**Table 1: Percent Inhibition of CCR2 Receptors**

| Rosemary Leaf Extract (µg/mL) | Caraway Seed Extract (µg/mL) | | |
|---|---|---|---|
| | 0 | 175 | 350 |
| 0 | Untreated | 10% | 25% |
| 5 | 4% | 28% | 53% |
| 10 | 18% | 50% | 66% |
| 20 | 41% | 64% | 79% |

As seen from the above table, all tested combinations of *Rosmarinus officinalis* leaf extract and *Carum carvi* seed extract demonstrate a synergistic effect in terms of CCR2 receptor inhibition (>1-fold increase from additive levels). The synergy was particularly pronounced in the combinations of 5 and 10 µg/mL of *Rosmarinus officinalis* leaf extract with 175µg/mL or 350µg/mL *Carum carvi* seed extract (>1.5-fold increase from additive levels). The synergistic effect was most pronounced with 5µg/mL of *Rosmarinus officinalis* leaf extract in combination with 175 µg/mL of *Carum carvi* seed extract (2-fold increase from additive levels).

One way these results are surprising is the relatively small amount of extract needed to see the synergistic effect. For example, at 175 µg/mL of *Carum carvi* seed extract alone (without *Rosmarinus officinalis* leaf extract), there was a 10% inhibition of the CCR2 receptors. Even doubling the amount of *Carum carvi* seed extract to 350 µg/mL exhibited only 25% inhibition. However, by adding just 5 µg/mL *Rosmarinus officinalis* leaf extract, the percentage jumped to 28% when the concentration of *Carum carvi* seed extract was maintained at 175 µg/mL. The results are particularly surprising given that at a concentration of 5 µg/mL *Rosmarinus officinalis* leaf extract alone (without *Carum carvi* seed extract extract), there was a mere 4% inhibition of the receptors. This means even at concentrations where the *Rosmarinus officinalis* leaf extract has very little activity on its own, its presence alone greatly boosts the inhibition of the *Carum carvi* seed extract.

Based on the demonstrated inhibition of the CCR2 receptors, it was unexpectedly found that the combination of *Rosmarinus officinalis* leaf and *Carum carvi* seed extracts outperforms (and in some instances greatly outperforms) either extract alone. Accordingly, the synergistic combination of *Rosmarinus officinalis* leaf and *Carum carvi* seed extracts may be effective to treat, reduce and/or ameliorate inflammation, particularly where the inflammation is CCR2 receptor-mediated inflammation.

### EXAMPLE 2: Effect on IL-6 Cytokine Release

*Rosmarinus officinalis* leaf extract and *Carum carvi* seed extract were tested alone and in combinations of varying concentrations and evaluated for reduction of IL-6 cytokine release according to Assay 2 above. IL-6 is an interleukin which acts as a pro-inflammatory cytokine. Reduction of IL-6 release is thus associated with a reduction in inflammation. The results of the experiment are shown in Table 2 below.

**Table 2: Percent Reduction of IL-6 Release**

| Rosemary Leaf Extract (µg/mL) | Caraway Seed Extract (µg/mL) | |
|---|---|---|
| | 0 | 50 |
| 0 | Untreated | 1% |
| 0.5 | -11% | 58% |
| 5 | -5% | 63% |
| 25 | 69% | 96% |

As seen from the above table, all tested combinations of *Rosmarinus officinalis* leaf extract and *Carum carvi* seed extract demonstrate a synergistic effect in terms of IL-6 reduction (>1-fold increase from additive levels). The synergistic effect was particularly pronounced with the combinations of 0.5 and 5 µg/mL of *Rosmarinus officinalis* leaf extract with 50 µg/mL of *Carum carvi* seed extract.

One way these results are surprising is the fact that most of the tested concentrations *Carum carvi* seed extract and *Rosmarinus officinalis* leaf extract had almost no, or even negative, reduction of IL-6 release when tested alone. Yet, these same concentrations jumped to over 50% percent reduction when the two extracts were combined. For example, at 50 µg/mL of *Carum carvi* seed extract (without *Rosmarinus officinalis* leaf extract), there was only a 1% reduction in IL-6 release. Moreover, at 0.5 and 0.5 µg/mL of *Rosmarinus officinalis* leaf extract (without *Carum carvi* seed extract), the results actually showed a negative percent reduction. The additive effect expected from the combinations of these concentrations would still be negative. Yet when combined, the percent reduction rose to 58% (0.5 µg/mL of *Rosmarinus officinalis* leaf extract with 50 µg/mL of *Carum carvi* seed extract) and 63% (5 µg/mL of *Rosmarinus officinalis* leaf extract with 50 µg/mL of *Carum carvi* seed extract). This means even at concentrations where the *Rosmarinus officinalis* leaf extract and *Carum carvi* seed extract have very little (if any) activity on their own, it is the presence of both extracts which results in the desired property of reduction of IL-6 release. Even at 25 µg/mL of *Rosmarinus officinalis* leaf extract, which exhibited a 69% reduction in IL-6 release, the number was increased to just under 100% when combined with 50 µg/mL of *Carum carvi* seed extract.

Based on the demonstrated reduction of IL-6 release, it was unexpectedly found that the combination of *Rosmarinus officinalis* leaf and *Carum carvi* seed extracts outperforms (and in some instances greatly outperforms) either extract alone. Accordingly, the synergistic combination of *Rosmarinus officinalis* leaf and *Carum carvi* seed extracts may be effective to treat, reduce and/or ameliorate inflammation, particularly inflammation related to IL-6 cytokine release.

### EXAMPLE 3: Effect on IL-8 Cytokine Release

*Rosmarinus officinalis* leaf extract and *Carum carvi* seed extract were tested alone and in combinations of varying concentrations and evaluated for reduction of IL-8 cytokine release according to Assay 2 above. IL-8 is a chemoattractant cytokine associated with inflammation. Reduction of IL-8 release is thus associated with a reduction in inflammation. The results of the experiment are shown in Table 3 below.

**Table 3: Percent Reduction of IL-8 Release**

| Rosemary Leaf Extract (µg/mL) | Caraway Seed Extract (µg/mL) | |
|---|---|---|
| | 0 | 50 |
| 0 | Untreated | 37% |
| 0.5 | 14% | 79% |
| 5 | 34% | 80% |
| 25 | 87% | 95% |

As seen from the above table, all tested combinations of *Rosmarinus officinalis* leaf extract and *Carum carvi* seed extract demonstrate a synergistic effect in terms of IL-8 reduction release (>1-fold increase from additive levels). The synergistic effect was particularly pronounced with the combination of 0.5 µg/mL of *Rosmarinus officinalis* leaf extract with 50 µg/mL of *Carum carvi* seed extract (>1.5-fold increase from additive levels).

One way these results are surprising is the relatively small amount of extract needed to see the synergistic effect. For example, at 50 µg/mL of *Carum carvi* seed extract (without *Rosmarinus officinalis* leaf extract), the percent reduction was 37%. When combined with just 0.5 µg/mL of *Rosmarinus officinalis* leaf extract, the amount jumped to 79%, even though that same concentration of *Rosmarinus officinalis* leaf extract exhibited only a modest percent reduction of 14% when used alone. This means even at concentrations where the *Rosmarinus officinalis* leaf extract has a modest activity on its own, its presence alone greatly boosts the inhibition of the *Carum carvi* seed extract.

Based on the demonstrated reduction of IL-8 release, it was unexpectedly found that the combination of *Rosmarinus officinalis* leaf and *Carum carvi* seed extracts outperforms (and in some instances greatly outperforms) either extract alone. Accordingly, the synergistic combination of *Rosmarinus officinalis* leaf and *Carum carvi* seed extracts may be effective to treat, reduce and/or ameliorate inflammation, particularly inflammation related to IL-8 cytokine release.

## Claims

1. A composition for use in a method of treating or preventing inflammatory disorders, the method comprising:
administering to a patient in need thereof a therapeutically effective dose of the composition, wherein the composition comprises *Carum carvi* seed extract and *Rosmarinus officinalis* extract,
wherein the *Carum carvi* seed extract is present at a concentration of about 50 to about 350 µg/mL, and the *Rosmarinus officinalis* extract is a *Rosmarinus officinalis* leaf extract present at a concentration of about 0.5 to about 25 µg/mL; wherein
the *Carum carvi* seed extract and *Rosmarinus officinalis* leaf extract each independently comprise polar extracts; and wherein
the polar extract was extracted using one or more of C₁-C₈ alcohols, C₁-Cs polyols, C₁-C₈ glycols and combinations of two or more thereof.

2. The composition for use according to claim 1, wherein the inflammatory disorder is arthritis, bronchitis, contact dermatitis, atopic dermatitis, psoriasis, seborrheic dermatitis, sumac and poison oak dermatitis, eczema, allergic dermatitis, polymorphous light eruptions, inflammatory dermatoses, folliculitis, alopecia, or acne inflammation.

3. The composition for use according to any of claims 1-2, wherein the extract is prepared using a solvent comprising methanol, ethanol, or a combination thereof with or without presence of water.

4. The composition for use according to any of claims 1-3, wherein the composition further comprises a pharmaceutically and/or cosmetically acceptable carrier selected from the group consisting of surfactants, chelating agents, emollients, humectants, conditioners, preservatives, opacifiers, fragrances, and combinations of two or more thereof.

5. The composition for use according to any of claims 1-4, wherein the composition is administered in the form of a solution, suspension, lotion, cream, serum, gel, stick, spray, ointment, liquid wash, soap bar, shampoo, hair conditioner, paste, foam, powder, mousse, shaving cream, hydrogel, or film-forming product.

6. The composition for use according to any of claims 1-5, wherein the *Carum carvi* seed extract is present at a concentration of about 50 µg/mL and the *Rosmarinus officinalis* leaf extract is present at a concentration of about 0.5 to about 5 µg/mL.

7. The composition for use according to claim 1 wherein the composition comprises a pharmaceutically and/or cosmetically acceptable carrier,
wherein the *Carum carvi* seed extract and *Rosmarinus officinalis* leaf extract are each independently aqueous alcohol extracts, and
wherein the composition is in the form of a solution, suspension, emulsion, lotion, cream, serum, gel, stick, spray, ointment, liquid wash, soap bar, shampoo, hair conditioner, paste, foam, powder, mousse, shaving cream, hydrogel, or film-forming product.

8. The composition for use according to claim 7, wherein the *Carum carvi* seed extract is present at a concentration of about 50 µg/mL and the *Rosmarinus officinalis* leaf extract is present at a concentration of about 0.5 to about 5 µg/mL.

9. The composition for use according to any of claims 1-8, wherein the *Carum carvi* seed extract and *Rosmarinus officinalis* leaf extract are each independently substantially free of essential oils.

10. The composition for use according to any of claims 1-9, wherein the *Carum carvi* seed extract and *Rosmarinus officinalis* extract are present in the composition at a combined concentration of about 0.1 to about 20 wt.% by weight of the total composition.

11. The composition for use according to any of claims 1-10, wherein the *Rosmarinus officinalis* extract and *Carum carvi* seed extract are present at a weight ratio of about 1:2 to about 1:100.

12. The composition for use according to any of claims 1-11, wherein the composition further comprises a pharmaceutically and/or cosmetically acceptable carrier selected from the group consisting of surfactants, chelating agents, emollients, humectants, conditioners, preservatives, opacifiers, fragrances, and combinations of two or more thereof.

13. The composition for use according to any of claims 1-12, wherein the composition is applied to a surface of skin of the patient,
or wherein the composition is applied to a mucous membrane of the patient.

14. *Carum carvi* seed extract for use in a method of treating or preventing inflammatory disorders, wherein the *Carum carvi* seed extract is administered to a patient in need thereof in combination with *Rosmarinus officinalis* extract in a composition,
wherein the *Carum carvi* seed extract is present in the composition at a concentration of about 50 to about 350 µg/mL, and the *Rosmarinus officinalis* extract is a *Rosmarinus officinalis* leaf extract present in the composition at a concentration of about 0.5 to about 25 µg/mL; wherein
the *Carum carvi* seed extract and *Rosmarinus officinalis* leaf extract each independently comprise polar extracts; and wherein
the polar extract was extracted using one or more of C₁-C₈ alcohols, C₁-Cs polyols, C₁-C₈ glycols and combinations of two or more thereof.

15. *Rosmarinus officinalis* extract for use in a method of treating or preventing inflammatory disorders, wherein the *Rosmarinus officinalis* extract is administered to a patient in need thereof in combination with *Carum carvi* seed extract in a composition,
wherein the *Carum carvi* seed extract is present in the composition at a concentration of about 50 to about 350 µg/mL, and the *Rosmarinus officinalis* extract is a *Rosmarinus officinalis* leaf extract present in the composition at a concentration of about 0.5 to about 25 µg/mL; wherein
the *Carum carvi* seed extract and *Rosmarinus officinalis* leaf extract each independently comprise polar extracts; and wherein
the polar extract was extracted using one or more of C₁-C₈ alcohols, C₁-Cs polyols, C₁-C₈ glycols and combinations of two or more thereof.

## Patentansprüche

1. Zusammensetzung zur Verwendung bei einem Verfahren zur Behandlung oder Vorbeugung von entzündlichen Erkrankungen, wobei das Verfahren Folgendes umfasst:
Verabreichen einer therapeutisch wirksamen Dosis der Zusammensetzung an einen Patienten, der dies benötigt, wobei die Zusammensetzung *Carum* carvi-Samenextrakt und *Rosmarinus officinalis*-Extrakt umfasst,
wobei der *Carum* carvi-Samenextrakt in einer Konzentration von etwa 50 bis etwa 350 µg/ml vorliegt und es sich bei dem *Rosmarinus officinalis*-Extrakt um einen *Rosmarinus* officinalis-Blattextrakt handelt, der in einer Konzentration von etwa 0,5 bis etwa 25 µg/ml vorliegt;
wobei
der *Carum* carvi-Samenextrakt und der *Rosmarinus officinalis*-Blattextrakt jeweils unabhängig polare Extrakte umfassen; und wobei
der polare Extrakt unter Verwendung von einem oder mehreren von C₁-C₈-Alkoholen, C₁-C₈-Polyolen, C₁-C₈-Glykolen und Kombinationen von zwei oder mehr davon extrahiert wurde.

2. Zusammensetzung zur Verwendung nach Anspruch 1, wobei es sich bei der entzündlichen Erkrankung um Arthritis, Bronchitis, Kontaktdermatitis, atopische Dermatitis, Psoriasis, seborrhoische Dermatitis, Sumach- und Gifteiche-Dermatitis, Ekzem, allergische Dermatitis, polymorphe Lichtdermatose, entzündliche Dermatosen, Follikulitis, Alopezie oder Akne-Entzündung handelt.

3. Zusammensetzung zur Verwendung nach einem der Ansprüche 1-2, wobei der Extrakt unter Verwendung eines Methanol, Ethanol oder eine Kombination davon umfassenden Lösungsmittels mit oder ohne Gegenwart von Wasser hergestellt wird.

4. Zusammensetzung zur Verwendung nach einem der Ansprüche 1-3, wobei die Zusammensetzung ferner einen pharmazeutisch und/oder kosmetisch unbedenklichen Träger umfasst, der aus der Gruppe bestehend aus Tensiden, Chelatbildnern, Weichmachern, Feuchthaltemitteln, Conditionern, Konservierungsmitteln, Trübungsmitteln, Duftstoffen und Kombinationen von zwei oder mehr davon ausgewählt ist.

5. Zusammensetzung zur Verwendung nach einem der Ansprüche 1-4, wobei die Zusammensetzung in Form einer Lösung, Suspension, Lotion, Creme, eines Serums, Gels, Stifts, Sprays, einer Salbe, Waschflüssigkeit, eines Seifenstücks, Shampoos, Haar-Conditioners, einer Paste, eines Schaums, Pulvers, einer Mousse, Rasiercreme, eines Hydrogels oder filmbildenden Produkts verabreicht wird.

6. Zusammensetzung zur Verwendung nach einem der Ansprüche 1-5, wobei der *Carum carvi*-Samenextrakt in einer Konzentration von etwa 50 µg/ml und der *Rosmarinus officinalis*-Blattextrakt in einer Konzentration von etwa 0,5 bis etwa 5 µg/ml vorliegt.

7. Zusammensetzung zur Verwendung nach Anspruch 1, wobei die Zusammensetzung einen pharmazeutisch und/oder kosmetisch unbedenklichen Träger umfasst,
wobei es sich bei dem *Carum carvi-*Samenextrakt und dem *Rosmarinus officinalis*-Blattextrakt jeweils unabhängig um wässrige Alkoholextrakte handelt und
wobei die Zusammensetzung in Form einer Lösung, Suspension, Emulsion, Lotion, Creme, eines Serums, Gels, Stifts, Sprays, einer Salbe, Waschflüssigkeit, eines Seifenstücks, Shampoos, Haar-Conditioners, einer Paste, eines Schaums, Pulvers, einer Mousse, Rasiercreme, eines Hydrogels oder filmbildenden Produkts vorliegt.

8. Zusammensetzung zur Verwendung nach Anspruch 7, wobei der *Carum carvi*-Samenextrakt in einer Konzentration von etwa 50 µg/ml und der *Rosmarinus officinalis-*Blattextrakt in einer Konzentration von etwa 0,5 bis etwa 5 µg/ml vorliegt.

9. Zusammensetzung zur Verwendung nach einem der Ansprüche 1-8, wobei der *Carum carvi*-Samenextrakt und der *Rosmarinus officinalis*-Blattextrakt jeweils unabhängig im Wesentlichen frei von ätherischen Ölen sind.

10. Zusammensetzung zur Verwendung nach einem der Ansprüche 1-9, wobei der *Carum* carvi-Samenextrakt und der *Rosmarinus officinalis-*Extrakt in der Zusammensetzung in einer kombinierten Konzentration von etwa 0,1 bis etwa 20 Gew.-% der Gesamtzusammensetzung vorliegen.

11. Zusammensetzung zur Verwendung nach einem der Ansprüche 1-10, wobei der *Rosmarinus officinalis*-Extrakt und der *Carum carvi*-Samenextrakt in einem Gewichtsverhältnis von etwa 1:2 bis etwa 1:100 vorliegen.

12. Zusammensetzung zur Verwendung nach einem der Ansprüche 1-11, wobei die Zusammensetzung ferner einen pharmazeutisch und/oder kosmetisch unbedenklichen Träger umfasst, der aus der Gruppe bestehend aus Tensiden, Chelatbildnern, Weichmachern, Feuchthaltemitteln, Conditionern, Konservierungsmitteln, Trübungsmitteln, Duftstoffen und Kombinationen von zwei oder mehr davon ausgewählt ist.

13. Zusammensetzung zur Verwendung nach einem der Ansprüche 1-12, wobei die Zusammensetzung auf eine Hautoberfläche des Patienten aufgebracht wird oder wobei die Zusammensetzung auf eine Schleimhaut des Patienten aufgebracht wird.

14. *Carum carvi*-Samenextrakt zur Verwendung bei einem Verfahren zur Behandlung oder Vorbeugung von entzündlichen Erkrankungen, wobei der *Carum carvi-*Samenextrakt einem diesen benötigenden Patienten in Kombination mit *Rosmarinus officinalis-*Extrakt in einer Zusammensetzung verabreicht wird,
wobei der *Carum carvi-*Samenextrakt in der Zusammensetzung in einer Konzentration von etwa 50 bis etwa 350 µg/ml vorliegt und es sich bei dem *Rosmarinus officinalis-*Extrakt um einen *Rosmarinus officinalis*-Blattextrakt handelt, der in der Zusammensetzung in einer Konzentration von etwa 0,5 bis etwa 25 µg/ml vorliegt; wobei
der *Carum carvi*-Samenextrakt und der *Rosmarinus officinalis-*Blattextrakt jeweils unabhängig polare Extrakte umfassen; und wobei
der polare Extrakt unter Verwendung von einem oder mehreren von C₁-C₈-Alkoholen, C₁-C₈-Polyolen, C₁-C₈-Glykolen und Kombinationen von zwei oder mehr davon extrahiert wurde.

15. *Rosmarinus officinalis-*Extrakt zur Verwendung bei einem Verfahren zur Behandlung oder Vorbeugung von entzündlichen Erkrankungen, wobei der *Rosmarinus officinalis*-Extrakt einem diesen benötigenden Patienten in Kombination mit *Carum* carvi-Samenextrakt in einer Zusammensetzung verabreicht wird,
wobei der *Carum carvi*-Samenextrakt in der Zusammensetzung in einer Konzentration von etwa 50 bis etwa 350 µg/ml vorliegt und es sich bei dem *Rosmarinus officinalis-*Extrakt um einen *Rosmarinus* officinalis-Blattextrakt handelt, der in der Zusammensetzung in einer Konzentration von etwa 0,5 bis etwa 25 µg/ml vorliegt; wobei
der *Carum carvi*-Samenextrakt und der *Rosmarinus officinalis*-Blattextrakt jeweils unabhängig polare Extrakte umfassen; und wobei
der polare Extrakt unter Verwendung von einem oder mehreren von C₁-C₈-Alkoholen, C₁-C₈-Polyolen, C₁-C₈-Glykolen und Kombinationen von zwei oder mehr davon extrahiert wurde.

## Revendications

1. Composition pour utilisation dans un procédé de traitement ou de prévention de troubles inflammatoires, le procédé comprenant :
l'administration à un patient qui en a besoin d'une dose thérapeutiquement efficace de la composition, la composition comprenant de l'extrait de graines de Carum carvi et de l'extrait de Rosmarinus officinalis,
dans laquelle l'extrait de graines de Carum carvi est présent à une concentration d'environ 50 à environ 350 µg/mL, et l'extrait de Rosmarinus officinalis est un extrait de feuilles de Rosmarinus officinalis présent à une concentration d'environ 0,5 à environ 25 µg/mL ; dans laquelle
l'extrait de graines de Carum carvi et l'extrait de feuilles de Rosmarinus officinalis comprennent chacun indépendamment des extraits polaires ; et dans laquelle l'extrait polaire a été extrait au moyen d'un ou plusieurs parmi des alcools en C₁-C₈, des polyols en C₁-C₈, des glycols en C₁-C₈ et des combinaisons de deux ou plus de ceux-ci.

2. Composition pour utilisation selon la revendication 1, dans laquelle le trouble inflammatoire est l'arthrite, la bronchite, la dermatite de contact, la dermatite atopique, le psoriasis, la dermatite séborrhéique, la dermatite au sumac et au sumac vénéneux, l'eczéma, la dermatite allergique, les éruptions cutanées polymorphes, les dermatoses inflammatoires, la folliculite, l'alopécie ou l'inflammation de l'acné.

3. Composition pour utilisation selon l'une quelconque des revendications 1 et 2, dans laquelle l'extrait est préparé en utilisant un solvant comprenant du méthanol, de l'éthanol ou une combinaison de ceux-ci avec ou sans présence d'eau.

4. Composition pour utilisation selon l'une quelconque des revendications 1 à 3, dans laquelle la composition comprend en outre un support pharmaceutiquement et/ou cosmétiquement acceptable choisi dans le groupe constitué des tensioactifs, des agents chélatants, des émollients, des humectants, des conditionneurs, des conservateurs, des agents opacifiants, des fragrances et des combinaisons de deux ou plus de ceux-ci.

5. Composition pour utilisation selon l'une quelconque des revendications 1 à 4, dans laquelle la composition est administrée sous la forme d'une solution, une suspension, une lotion, une crème, un sérum, un gel, un bâton, un spray, une pommade, un liquide de lavage, un pain de savon, un shampoing, un après-shampoing, une pâte, une mousse, une poudre, une mousse à raser, un hydrogel ou un produit filmogène.

6. Composition pour utilisation selon l'une quelconque des revendications 1 à 5, dans laquelle l'extrait de graines de Carum carvi est présent à une concentration d'environ 50 µg/mL et l'extrait de feuilles de Rosmarinus officinalis est présent à une concentration d'environ 0,5 à environ 5 µg/mL.

7. Composition pour utilisation selon la revendication 1, dans laquelle la composition comprend un support pharmaceutiquement et/ou cosmétiquement acceptable,
dans laquelle l'extrait de graines de Carum carvi et l'extrait de feuilles de Rosmarinus officinalis sont chacun indépendamment des extraits alcooliques aqueux, et
dans laquelle la composition se présente sous la forme d'une solution, une suspension, une émulsion, une lotion, une crème, un sérum, un gel, un bâton, un spray, une pommade, un liquide de lavage, un pain de savon, un shampoing, un après-shampoing, une pâte, une mousse, une poudre, une mousse à raser, un hydrogel ou un produit filmogène.

8. Composition pour utilisation selon la revendication 7, dans laquelle l'extrait de graines de Carum carvi est présent à une concentration d'environ 50 µg/mL et l'extrait de feuilles de Rosmarinus officinalis est présent à une concentration d'environ 0,5 à environ 5 µg/mL.

9. Composition pour utilisation selon l'une quelconque des revendications 1 à 8, dans laquelle l'extrait de graines de Carum carvi et l'extrait de feuilles de Rosmarinus officinalis sont chacun indépendamment sensiblement exempts d'huiles essentielles.

10. Composition pour utilisation selon l'une quelconque des revendications 1 à 9, dans laquelle l'extrait de graines de Carum carvi et l'extrait de Rosmarinus officinalis sont présents dans la composition à une concentration combinée d'environ 0,1 à environ 20 % en poids par rapport au poids total de la composition.

11. Composition pour utilisation selon l'une quelconque des revendications 1 à 10, dans laquelle l'extrait de Rosmarinus officinalis et l'extrait de graines de Carum carvi sont présents dans un rapport pondéral d'environ 1:2 à environ 1:100.

12. Composition pour utilisation selon l'une quelconque des revendications 1 à 11, dans laquelle la composition comprend en outre un support pharmaceutiquement et/ou cosmétiquement acceptable choisi dans le groupe constitué des tensioactifs, des agents chélatants, des émollients, des humectants, des conditionneurs, des conservateurs, des agents opacifiants, des fragrances et des combinaisons de deux ou plus de ceux-ci.

13. Composition pour utilisation selon l'une quelconque des revendications 1 à 12, dans laquelle la composition est appliquée sur une surface de la peau du patient,
ou dans laquelle la composition est appliquée sur une muqueuse du patient.

14. Extrait de graines de Carum carvi pour utilisation dans un procédé de traitement ou de prévention de troubles inflammatoires, dans lequel l'extrait de graines de Carum carvi est administré à un patient qui en a besoin en combinaison avec l'extrait de Rosmarinus officinalis dans une composition,
dans lequel l'extrait de graines de Carum carvi est présent dans la composition à une concentration d'environ 50 à environ 350 µg/mL, et l'extrait de Rosmarinus officinalis est un extrait de feuilles de Rosmarinus officinalis présent dans la composition à une concentration d'environ 0,5 à environ 25 µg/mL ; dans lequel
l'extrait de graines de Carum carvi et l'extrait de feuilles de Rosmarinus officinalis comprennent chacun indépendamment des extraits polaires ; et dans lequel
l'extrait polaire a été extrait au moyen d'un ou plusieurs parmi des alcools en C₁-C₈, des polyols en C₁-C₈, des glycols en C₁-C₈ et des combinaisons de deux ou plus de ceux-ci.

15. Extrait de Rosmarinus officinalis pour utilisation dans un procédé de traitement ou de prévention de troubles inflammatoires, dans lequel l'extrait de Rosmarinus officinalis est administré à un patient qui en a besoin en combinaison avec l'extrait de graines de Carum carvi dans une composition,
dans lequel l'extrait de graines de Carum carvi est présent dans la composition à une concentration d'environ 50 à environ 350 µg/mL, et l'extrait de Rosmarinus officinalis est un extrait de feuilles de Rosmarinus officinalis présent dans la composition à une concentration d'environ 0,5 à environ 25 µg/mL ; dans lequel
l'extrait de graines de Carum carvi et l'extrait de feuilles de Rosmarinus officinalis comprennent chacun indépendamment des extraits polaires ; et dans lequel
l'extrait polaire a été extrait au moyen d'un ou plusieurs parmi des alcools en C₁-C₈, des polyols en C₁-C₈, des glycols en C₁-C₈ et des combinaisons de deux ou plus de ceux-ci.
